# EUROPEAN PATENT SPECIFICATION

(11) **EP 4 338 793 B1**
(45) Date of publication and mention of the grant of the patent: **10.06.2026**
(21) Application number: 22807575.0
(22) Date of filing: 04.01.2022
(51) Int. Cl.: A61B 18/14, A61B 18/00, A61B 17/00

(54) **HIGH-FREQUENCY TREATMENT HANDPIECE**
HOCHFREQUENZ-BEHANDLUNGSHANDSTÜCK
PIÈCE À MAIN DE TRAITEMENT À HAUTE FRÉQUENCE

(30) Priority: 10.05.2021 KR 20210059954
(43) Date of publication of application: 20.03.2024
(73) Proprietor: Tentech Inc., Seoul 06103 (KR)
(72) Inventor: KIM, Ki Hang, Seoul 04799 (KR); CHOI, Min Ji, Seoul 04799 (KR)
(74) Representative: Gille Hrabal Partnerschaftsgesellschaft mbB Patentanwälte
(86) International application number: PCT/KR2022/000103
(87) International publication number: WO 2022/239925

(56) References cited:
- KR-A- 20050 114 676
- KR-A- 20050 114 676
- KR-A- 20200 028 112
- KR-A- 20200 085 450
- KR-A- 20200 085 450
- KR-B1- 101 290 606
- US-A1- 2004 111 087
- US-A1- 2010 016 849

## Description

### Technical Field

The present disclosure relates to a radio frequency treatment handpiece. More particularly, the present disclosure relates to a handpiece structure in which heat from various components, including electrodes, is removed by supplying a low-temperature gaseous refrigerant to prevent bums when radio frequency waves are generated on skin and allowing the gaseous refrigerant that has undergone heat exchange to flow inside the handpiece.

### Background Art

Human skin consists of layers of the stratum corneum, epidermis, dermis, and hypodermis, and the functions of the skin deteriorate due to aging and the action of ultraviolet rays.

The epidermis, which is the most superficial layer of the skin, is subdivided into several layers including the stratum corneum, stratum lucidum, stratum granulosum, stratum spinosum, and stratum basale depending on location and function, and plays important roles in protection, defense, and secretion.

The dermis is located beneath the epidermis and adjacent to the stratum basale, making up most of the skin. The dermis is composed of two layers: the papillary layer which contains moisture, proteins, carbohydrates, mucopolysaccharides, minerals, and inorganic salts in a jelly state and where capillaries related to blood circulation and lymphatic vessels carrying lymph are located; and the reticular layer which contains collagen, collagen fibers associated with skin wrinkles, elastin, elastic fibers that give skin elasticity, and a matrix (water reservoir).

In addition, the hypodermis is located between the dermis and muscles and bones, contains a large amount of fat, and constitutes the lowest layer of the skin. The hypodermis spreads evenly throughout the body, serves to maintain elasticity and acts as a buffer, absorbing shock to prevent internal damage, as well as preventing heat loss and maintaining body temperature.

Meanwhile, as interest in skin increases, a variety of skin care devices related to skin treatments are being developed, and at the same time, many cosmetic dermatology hospitals and skin treatment clinics are emerging.

The most widely known skin treatment method recently is radio frequency (RF) therapy, in which radio frequency energy is applied to skin to induce local thermal damage within the skin to create microscopic wounds in order to promote skin regeneration from the epithelium to the dermis, and the microscopic wounds stimulate the release of cell growth factors to maximize natural healing and regeneration of the skin.

The above-described method delivers high heat energy to the skin by means of radio frequency, and thus there is a possibility that burns may occur in the epidermis. That is why a refrigerant is provided to a treatment area to cool the area to prevent burns. However, when water or a liquid refrigerant is used, refrigerant waste can be a problem because the refrigerant used for cooling is used only once and then discarded. Moreover, when water or a liquid refrigerant is used, a handpiece becomes heavy, putting a burden on the hands and wrists of an operator when performing a procedure for a long time.

On the other hand, in the case of radio frequency devices, when radio frequency waves are generated, considerable heat is produced not only in electrodes but also in components used to drive the handpiece. Generally, a separate cooling device such as a fan is installed and used in a handpiece to remove such heat. In this case as well, the problem is that the handpiece becomes heavy as described above. Publication KR 2020 0 085 450 A discloses an electrode cartridge for skin care treatment and a handpiece for skin care treatment having the same.

### Disclosure

### Technical Problem

The present disclosure has been made to solve the above problems, and an objective of the present disclosure is to provide a cooling structure of a handpiece that simultaneously cools a treatment target area of a recipient and cools the inside of the handpiece.

### Technical Solution

In order to achieve the above mentioned objective, there is provided a radio frequency treatment handpiece, including: a handpiece housing configured to have a first containing space therein and a circuit board installed in the first containing space so as to operate the handpiece, and have a frontside thereof being open; an electrode tip configured to have a nozzle portion that is detachably coupled to the handpiece housing and provided with an RF electrode for generating radio frequency waves by electric power applied thereto; an electrode assembly installed in the first containing space, provided with a valve supply channel that receives a gaseous refrigerant from a refrigerant supply means, a second containing space separate from the first containing space and a main body, and includes a power distributer for applying electric power to the electrode tip and a solenoid valve configured to communicate with the valve supply channel and to control flow of the gaseous refrigerant; and an actuator installed in a backside of the first containing space and coupled to the electrode assembly so as to vibrate the electrode assembly according to a control signal. The electrode assembly comprises:
a first body portion having a rear outlet that communicates the first containing space and the second containing space;
a nozzle coupling portion coupled to an inner peripheral surface of the nozzle portion so as to close an open area located inside the nozzle portion;
and a refrigerant supply channel configured to communicate with the valve supply channel so as to deliver a gaseous refrigerant to the electrode tip, and the solenoid valve is provided between the refrigerant supply channel and the valve supply channel so as to control flow of the gaseous refrigerant according to a control signal.

In the radio frequency treatment handpiece according to an embodiment of the present disclosure, at least one through hole may be formed in a front outer wall of the handpiece housing, and on a side of the first body portion of the electrode assembly, a stepped portion that is inserted and joined into a fixing groove formed in the nozzle portion and a pressing portion that protrudes through the through hole of the handpiece housing may be provided.

In the radio frequency treatment handpiece according to an embodiment of the present disclosure, the refrigerant supply channel may be fixed on a central axis of the first body portion and communicates with the valve supply channel, the electrode assembly may further include: a second body portion having a channel through hole through which refrigerant supply channel passes, and formed to partition the first containing space and the second containing space; and a second flange extending outward from an end of the second body portion, and the power distributer may be inserted into and fixed to the second body portion.

In the radio frequency treatment handpiece according to an embodiment of the present disclosure, the power distributer may be installed to be fixed on the refrigerant supply channel, and a plurality of electrode leads may be formed on a surface of the power distributer to be electrically connected to the RF electrode.

In the radio frequency treatment handpiece according to an embodiment of the present disclosure, the second flange may be provided with a plurality of through holes on corresponding positions of a plurality of electrode leads, and the plurality of electrode leads may each penetrate the plurality of through holes and contact the RF electrode.

In the radio frequency treatment handpiece according to an embodiment of the present disclosure, the electrode tip may further include: a distributer that uniformly distributes the gaseous refrigerant supplied from the refrigerant supply channel and supplies the gaseous refrigerant to the RF electrode; and a side outlet that communicates with the electrode assembly, so that the gaseous refrigerant that has exchanged heat with the RF electrode moves to the side outlet.

In the radio frequency treatment handpiece according to an embodiment of the present disclosure, a first outlet may be provided in an area of the first body portion to communicate with the side outlet and the second body portion, and a second outlet may be provided in the second body portion to communicate with the first outlet and the second containing space, so that the refrigerant heat exchange space, the second containing space, and the first containing space may be communicated in that order.

### Advantageous Effects

According to a handpiece according to an embodiment of the present disclosure, since cooling of the internal space of a handpiece is achieved in addition to overall cooling of a treatment target area, it is possible to provide a lightweight handpiece without a separate cooling device, thereby reducing the fatigue and burden on an operator's hands and wrists during the procedure, enabling long-time operation and use.

### Description of Drawings

FIG. 1 is a cross-sectional view showing a cross-section of a handpiece according to an embodiment of the present disclosure;
FIG. 2 is an enlarged cross-sectional view showing a cross-section of the front side of a handpiece according to an embodiment of the present disclosure;
FIG. 3 is a cross-sectional view showing the handpiece structure with an electrode tip removed according to an embodiment of the present disclosure;
FIG. 4 is a top view showing the top surface of an electrode assembly according to an embodiment of the present disclosure;
FIG. 5 is a perspective view showing the electrode assembly viewed from the rear side according to an embodiment of the present disclosure; and
FIG. 6 is a conceptual diagram showing a supply flow path of a gaseous refrigerant and the movement of the gaseous refrigerant that has undergone heat exchange into the internal space of the handpiece by the handpiece structure according to an embodiment of the present disclosure.

### Best Mode

Similar reference numbers in the drawings refer to identical or similar functions across various aspects.

Hereinafter, specific details for carrying out the present disclosure will be described based on embodiments with reference to the drawings, and these embodiments are described in sufficient detail to enable those skilled in the art to practice the present disclosure.

It should be understood that the various embodiments of the present disclosure are different from one another but are not necessarily mutually exclusive. For example, specific shapes, structures, and characteristics described herein with respect to one embodiment may be implemented in other embodiments without departing from the scope of the present disclosure.

Therefore, the detailed description described below is not intended to be taken in a limited sense, and the scope of the present disclosure is limited solely by the appended claims, together with all equivalents to what those claims assert if properly described.

A handpiece 100 according to an embodiment of the present disclosure includes a handpiece housing 110, an actuator 120, an electrode tip 130, and an electrode assembly 140.

### < Description of Components of Handpiece>

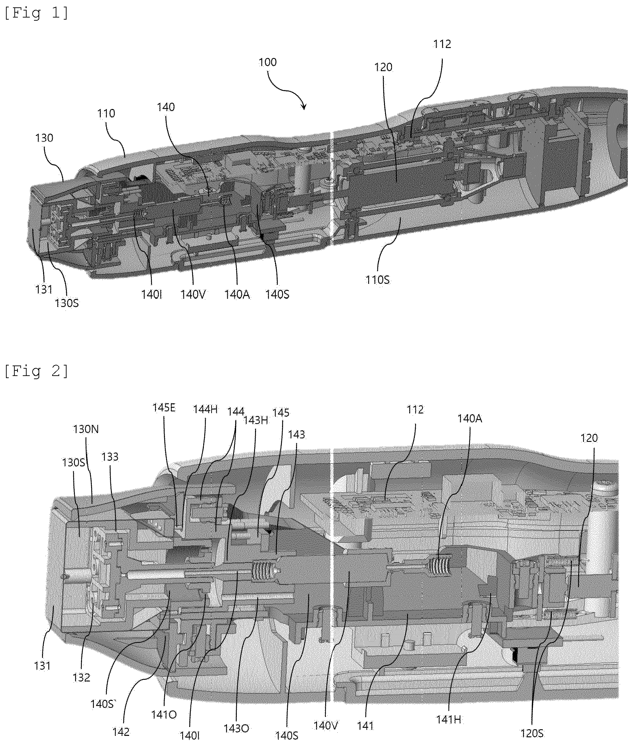

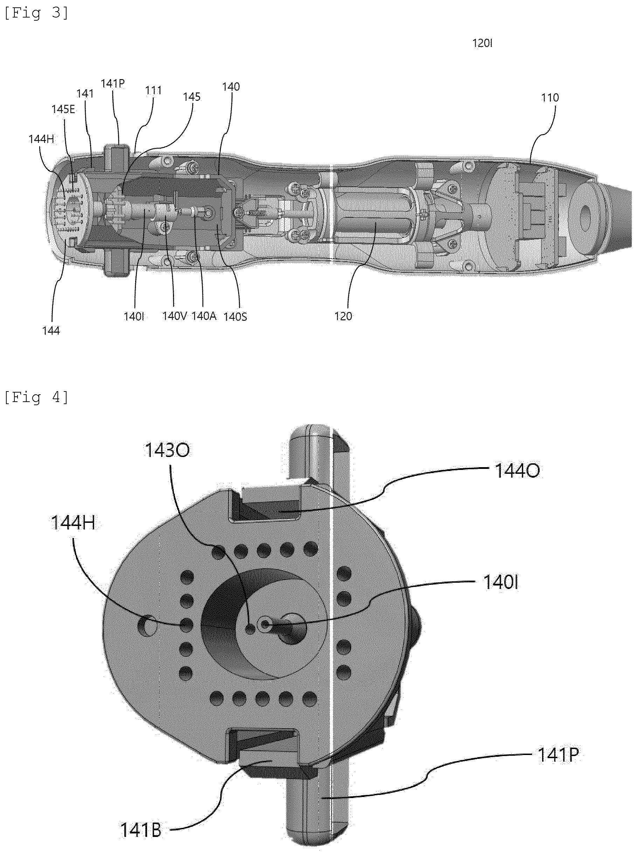

Hereinafter, the components of the handpiece 100 will be described with reference to FIGS. 1 to 5.

The handpiece housing 110 has an open front and has a first containing space 110S thereinside. The rear end of the first containing space 110S is connected to a cable (not shown) connected to a main body (not shown).

In addition, a circuit board 112 for operating the handpiece is installed inside the handpiece housing 110. The circuit board 112 is electrically connected to a plurality of wires, signal lines, etc. in the cable. The circuit board 112 is connected to various mechanical/electrical components such as the actuator 120, an RF electrode 131, or a solenoid valve 140V in the handpiece 100. Accordingly, various control signals such as a control signal for radio frequency generation, opening and closing control of the solenoid valve 140V, and vibration control for the electrode assembly 140 are received to control the overall operation of the handpiece.

The rear of the handpiece housing 110 is connected to the main body (not shown) through the cable.

In addition, at least one through hole 111 is formed on the outer wall of the front side of the handpiece housing 110. The through hole 111 is for combination with the electrode assembly 140, which will be described below.

Although the first containing space 110S of the handpiece housing 110 is not divided into front and rear, for convenience of explanation, the first containing space 110S will be described as being divided into a front containing space and a rear containing space.

The actuator 120 is installed in the rear area of the first containing space 110S. In addition, a drive shaft of the actuator 120 is coupled to the rear of the electrode assembly 140 so that the electrode assembly may be moved in the forward and backward directions. Meanwhile, one end of a restoration spring 120S is fixed to one surface of the handpiece housing 110, and the other end of the restoration spring 120S is fixed to the rear surface of the electrode assembly 140.

Accordingly, when the actuator 120 operates to move the electrode assembly 140 in the forward and backward directions, in response to the displacement of the electrode assembly 140, the restoration spring 120S restores the electrode assembly 140 to the original position thereof. That is, the electrode assembly 140 may be vibrated in the forward and backward directions due to displacement by the actuator 120 and restoration by the restoration spring 120S. At this time, the frequency may vary depending on the control signal used to operate the actuator 120.

The electrode tip 130 has a tapered tube-shaped nozzle portion 130N so as to be detachable from the handpiece housing 110. The side wall of the nozzle portion 130N is inserted to be at least partially positioned between the handpiece housing 110 and the electrode assembly 140. In addition, at least one RF electrode 131 that receives power and generates radio frequencies is installed to close the open surface of the nozzle portion 130N, and in this case, a predetermined distance is spaced between the back of the RF electrode 131 and a distributer 132 to form a predetermined refrigerant heat exchange space 130S. Meanwhile, when the front of the RF electrode 131 comes in contact with the skin surface, the RF electrode 131 is capacitively connected to the skin surface. Depending on implementation, the RF electrode 131 may be made of a single electrode or may be provided as a combination of multiple electrodes.

The distributer 132 is mechanically connected to a refrigerant supply channel 140I and fine channels are formed to provide a gaseous refrigerant to the entire rear area of the RF electrode 131. In general, when supplying gaseous refrigerant to the RF electrode 131 through a single flow path, since only the rear area of the RF electrode 131 located on the same axis as the refrigerant supply channel 140I is cooled intensively, a temperature gradient occurs in which the temperature difference becomes more severe as the distance from the central area of the RF electrode 131 increases. Thus, by providing the distributer 132, the entire area on the back of the RF electrode 131 is cooled evenly.

In addition, although not shown in the drawings, a flexible power distributer for uniformly providing power to the RF electrodes 131 or a plurality of electric pads corresponding to the number of RF electrodes 131 may be further provided on one side or inside the distributer 132. The back of the flexible power distributer or the back of the electric pads is electrically connected to an electrode lead 145E of a power distributer 145.

A side outlet 133 is a fine channel provided on the outside of the distributer 132 and communicates with the electrode assembly 140.

The electrode assembly 140 is a housing installed in front of the first containing space 110S of the handpiece housing 110, is formed with a second containing space 140S having a separate space from the containing space 110S, has a built-in power distributer for operating the handpiece, and a flow path is formed for the transfer of gaseous refrigerant.

The electrode assembly 140 is installed in front of the first containing space 110S to deliver gaseous refrigerant delivered through a soft refrigerant supply tube (not shown) in communication with the main body to the electrode tip 130 along a predetermined flow path.

The electrode assembly 140 has a structure formed integrally with a first body portion 141 and a nozzle coupling portion 142. In addition, a second body portion 143 and a second flange 144 are integrally formed and fixedly coupled to the first body portion 141. For convenience of explanation, each part will be divided into components.

It is preferable that the first body portion 141 has a rear outlet 141H formed in at least one area thereof that communicates with the first containing space 110S, and that the front side of the first body portion 141 is closed or first body portion 141 has a closed structure that ensures airtightness. As a result, the first containing space 110S and the second containing space 140S are separated. It is desirable that the two spaces communicate only through the rear outlet 141H so that a closed system in which gaseous refrigerant may circulate is formed inside the handpiece housing 110. In addition, a first outlet 1410 is provided on one area of the first body portion 141 communicating with the side outlet 133 of the electrode tip 130 and the second body portion 143.

The first body portion 141 may be deformed to some extent when pressed and is preferably made of a soft material such as electrically insulating plastic, but is not limited thereto.

Meanwhile, a stepped portion 141B and a pressing portion 141P are provided in the first body portion 141. The stepped portion 141B is formed on one side of the first body portion 141. The step portion 141B is inserted and fitted into a fixing groove formed in the nozzle portion 130N. The pressing portion 141P is also formed on one side of the first body portion 141, and the pressing portion 141P protrudes so as to penetrate a through hole 111 formed in the handpiece housing 110S.

Accordingly, when a recipient (a person receiving the treatment) presses the pressing portion 141P after finishing use, the first body portion 141 is deformed and the stepped portion 141B falls out from the fixing groove formed in the nozzle portion 130N. As a result, the electrode tip 130 is separated from the handpiece housing 110, and after separation, the recipient stops pressing the pressing portion 141P to restore the first body portion 141 to the initial state thereof.

The refrigerant supply channel 140I is provided on the second containing space 140S and is installed on the central axis of the first body portion 141. To be specific, the refrigerant supply channel 140I is installed through a channel through hole 143H formed in the longitudinal direction along the central axis of the second body portion 143, which will be described later. One side of the refrigerant supply channel 140I is in communication with the refrigerant heat exchange space 130S, and the other side of the refrigerant supply channel 140I is in mechanical communication with the solenoid valve 140V.

The solenoid valve 140V receives a valve opening/closing signal synchronized with a control signal for generating radio frequency from the main body (not shown) to generate radio frequency at the electrode tip 130 and open the solenoid valve 140V. One side of the solenoid valve 140V is mechanically communicated with the refrigerant supply channel 140I, and the other side of the solenoid valve 140V is mechanically communicated with the valve supply channel 140A.

Meanwhile, one side of the valve supply channel 140A is communicated with the solenoid valve 140V, and the other side of the valve supply channel 140A is mechanically communicated with the refrigerant supply tube (not shown) to receive gaseous refrigerant from the main body (not shown).

Accordingly, the cold gaseous refrigerant provided from the main body (not shown) is delivered to the solenoid valve 140V through the valve supply channel 140A, and the gaseous refrigerant that has passed through the solenoid valve 140V passes through the refrigerant supply channel 140I and is provided to the refrigerant heat exchange space 130S of the electrode tip 130.

The nozzle coupling portion 142 is coupled to the inner peripheral surface of the nozzle portion 130N to close the open area. A space is created between the electrode tip 130 and the first body portion 141 by the nozzle coupling portion 142, and this space is divided as a second containing space 140S'. Meanwhile, a space of the first body portion 141 located behind the second body portion 143 will be described by dividing the space as the previously described second containing space 140S. The reason for describing the two spaces separately is to provide convenience in explaining the flow of gaseous refrigerant later.

The second body portion 143 is installed on the first body portion 141 and is installed inside the second containing space (140S) and may be provided in a predetermined cylindrical shape. The channel through hole 143H is formed in the longitudinal direction along the central axis of the second body portion 143 so that the refrigerant supply channel 140I may be installed through the central axis of the second body portion 143. In addition, a second outlet 1430 communicating with the first outlet 1410 and the second containing space 140S is formed on the inner peripheral surface or the lower surface of the second body portion 143.

The second flange 144 extends from one end of the second body portion 143 in an outward direction. A plurality of through holes 144H are formed on the second flange 144 at positions corresponding to a plurality of electrode leads 145E. In addition, a predetermined open groove 1440 is formed in the second flange 144 so that the stepped portion 141B may be displaced in the direction of the central axis by the pressure of the pressing portion 141P.

The power distributer 145 is fixedly installed on the refrigerant supply channel 140I and is installed adjacent to the rear of the second flange 144. The plurality of electrode leads 145E are formed on one surface of the power distributer 145 and are electrically connected to the RF electrode 131 of the electrode tip 130. Preferably, the electrode leads 145E are provided to pass through the through holes 144H formed on the second flange 144. Accordingly, the electrode leads 145E may be electrically connected by contacting the back of the flexible power distributer or the back of the electric pads located in the distributer 132 of the electrode tip 130 described above.

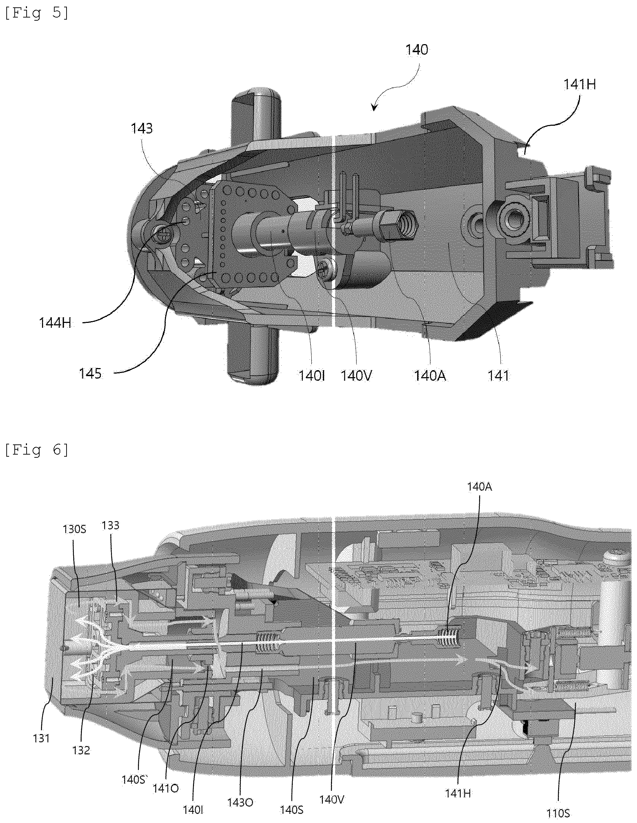

Hereinafter, the path along which the gaseous refrigerant flows will be briefly described with reference to FIG. 6.

### < Description of Flow Path of Gaseous Refrigerant>

With reference to FIG. 6, the flow path of the gaseous refrigerant will be described in order.
1. The gaseous refrigerant is supplied to the valve supply channel 140A by a refrigerant supply means (not shown) installed in the main body outside the handpiece 100. [main body-> valve supply channel 140A]
2. The valve supply channel 140A is mechanically communicated with the solenoid valve 140V, so that when the solenoid valve 140V is opened, the gaseous refrigerant is transferred from the valve supply channel 140A to the refrigerant supply channel 140I. [valve supply channel 140A-> solenoid valve 140V-> refrigerant supply channel 140I]
3.The refrigerant supply channel 140I is mechanically communicated with the refrigerant heat exchange space 130S of the electrode tip 130, and transfers the gaseous refrigerant passing through the solenoid valve 140V to the refrigerant heat exchange space 130S. [refrigerant supply channel 140I-> refrigerant heat exchange space 130S]
4. Heat exchange occurs when the gaseous refrigerant comes into contact with the back of the RF electrode 131.
5. The gaseous refrigerant that has undergone heat exchange passes through the side outlet 133 and is delivered to the second containing space 140S'. [refrigerant heat exchange space 130S-> side outlet 133-> second containing space 140S']
6. The gaseous refrigerant passing through the side outlet 133 is collected in the second containing space 140S, and the gaseous refrigerant is moved toward the second body portion 143 through the first outlet 1410. [second containing space 140S-> first outlet 1410-> second body portion 143]
7. The gaseous refrigerant is moved to the second containing space 140S through the second outlet 1430 provided in the second body portion 143. [second body portion 143-> second outlet 143O-> second containing space 140S]
8. The gaseous refrigerant moves to the rear outlet 141H provided in the first body portion 141, thereby cooling the actuator 120. [second containing space 140S-> rear outlet 141H-> actuator 120]

As described above, the present disclosure has been described with specific details such as specific components, limited embodiments, and drawings, but these are provided only to facilitate a more general understanding of the present disclosure, and the present disclosure is not limited to the above embodiments. Various modifications and variations may be made from the above description by those skilled in the art to which the present disclosure pertains.

Therefore, the scope of the present disclosure should not be limited to the described embodiments.

## Claims

1. A radio frequency treatment handpiece (100), comprising:
a handpiece housing (110) configured to have a first containing space (110S) therein and a circuit board (112) installed in the first containing space (110S) so as to operate the handpiece (100), and have a frontside thereof being open;
an electrode tip (130) configured to have a nozzle portion (130N) that is detachably coupled to the handpiece housing (110) and provided with an RF electrode (131) for generating radio frequency waves by electric power applied thereto;
an electrode assembly (140) installed in the first containing space (110S), provided with a valve supply channel (140I) that is configured to receive a gaseous refrigerant from a refrigerant supply means, a second containing space (140S) separate from the first containing space (110S) and a main body, and includes a power distributer (145) for applying electric power to the electrode tip (130) and a solenoid valve (140V) configured to communicate with the valve supply channel (140I) and to control flow of the gaseous refrigerant; and
an actuator (120) installed in a backside of the first containing space (110S) and coupled to the electrode assembly (140) so as to vibrate the electrode assembly (140) according to a control signal,
wherein the electrode assembly (140) comprises:
a first body portion (141) having a rear outlet (141H) that communicates the first containing space (110S) and the second containing space (140S);
a nozzle coupling portion (142) coupled to an inner peripheral surface of the nozzle portion (130N) so as to close an open area located inside the nozzle portion (130N); and
a refrigerant supply channel (140I) configured to communicate with the valve supply channel (140I) so as to deliver a gaseous refrigerant to the electrode tip (130), and
the solenoid valve (140V) is provided between the refrigerant supply channel (140I) and the valve supply channel (140I) so as to control flow of the gaseous refrigerant according to a control signal.

2. The radio frequency treatment handpiece (100) of claim 1, wherein at least one through hole (111) is formed in a front outer wall of the handpiece (100) housing (110), and on a side of the first body portion (141) of the electrode assembly (140), a stepped portion (141B) that is inserted and joined into a fixing groove formed in the nozzle portion (130N) and a pressing portion (141P) that protrudes through the through hole (111) of the handpiece housing (110) are provided.

3. The radio frequency treatment handpiece (100) of claim 1, wherein the refrigerant supply channel (140I) is fixed on a central axis of the first body portion (141) and communicates with the valve supply channel (140I),
the electrode assembly (140) further comprises: a second body portion (143) having a channel through hole (143H) through which refrigerant supply channel (140I) passes, and formed to partition the first containing space (110S) and the second containing space (140S); and a second flange (144) extending outward from an end of the second body portion (143), and
the power distributer (145) is inserted into and fixed to the second body portion (143).

4. The radio frequency treatment handpiece (100) of claim 3, wherein the power distributer (145) is installed to be fixed on the refrigerant supply channel (140I), and a plurality of electrode leads (145E) are formed on a surface of the power distributer (145) to be electrically connected to the RF electrode (131).

5. The radio frequency treatment handpiece (100) of claim 3, wherein the second flange (144) is provided with a plurality of through holes (111) on corresponding positions of a plurality of electrode leads (145E), and the plurality of electrode leads (145E) each penetrate the plurality of through holes (111) and contact the RF electrode (131).

6. The radio frequency treatment handpiece (100) of claim 3, wherein the electrode tip (130) further comprises: a distributer (132) that uniformly distributes the gaseous refrigerant supplied from the refrigerant supply channel (140I) and supplies the gaseous refrigerant to the RF electrode (131); and a side outlet (133) that communicates with the electrode assembly (140), so that the gaseous refrigerant that has exchanged heat with the RF electrode (131) moves to the side outlet (133).

7. The radio frequency treatment handpiece (100) of claim 6, wherein a first outlet (1410) is provided in an area of the first body portion (141) to communicate with the side outlet (133) and the second body portion (143), and a second outlet is provided in the second body portion (143) to communicate with the first outlet (1410) and the second containing space (140S), so that the refrigerant heat exchange space (130S), the second containing space (140S), and the first containing space (110S) are communicated in that order.

## Patentansprüche

1. Ein Radiofrequenz-Behandlungshandstück (100), umfassend:
ein Handstückgehäuse (110), das so konfiguriert ist, dass es einen ersten Aufnahmeraum (110S) darin und eine Leiterplatte (112) aufweist, die in dem ersten Aufnahmeraum (110S) installiert ist, um das Handstück (100) zu betreiben, und eine Vorderseite davon offen ist;
eine Elektrodenspitze (130), die so konfiguriert ist, dass sie einen Düsenabschnitt (130N) aufweist, der lösbar mit dem Handstückgehäuse (110) gekoppelt ist und mit einer HF-Elektrode (131) zum Erzeugen von Radiofrequenzwellen durch daran angelegte elektrische Leistung versehen ist;
eine in dem ersten Aufnahmeraum (110S) installierte Elektrodenanordnung (140), die mit einem Ventilversorgungskanal (140I), der so konfiguriert ist, dass er ein gasförmiges Kältemittel von einer Kältemittelversorgungseinrichtung empfängt, einem zweiten Aufnahmeraum (140S), der von dem ersten Aufnahmeraum (110S) getrennt ist, und einem Hauptkörper versehen ist, und einen Leistungsverteiler (145) zum Anlegen von elektrischer Leistung an die Elektrodenspitze (130) und ein Magnetventil (140V) enthält, das dazu eingerichtet ist, mit dem Ventilversorgungskanal (140I) in Verbindung zu stehen und einen Fluss des gasförmigen Kältemittels zu steuern; und
einen Aktuator (120), der an einer Rückseite des ersten Aufnahmeraums (110S) installiert und mit der Elektrodenanordnung (140) gekoppelt ist, um die Elektrodenanordnung (140) gemäß einem Steuersignal in Vibration zu versetzen,
wobei die Elektrodenanordnung (140) umfasst:
einen ersten Körperabschnitt (141) mit einem hinteren Auslass (141H), der den ersten Aufnahmeraum (110S) und den zweiten Aufnahmeraum (140S) verbindet;
einen Düsenkopplungsabschnitt (142), der mit einer inneren Umfangsfläche des Düsenabschnitts (130N) gekoppelt ist, um einen offenen Bereich zu schließen, der sich innerhalb des Düsenabschnitts (130N) befindet; und
einen Kältemittelversorgungskanal (140I), der so konfiguriert ist, dass er mit dem Ventilversorgungskanal (140I) in Verbindung steht, um ein gasförmiges Kältemittel an die Elektrodenspitze (130) zu liefern, und
wobei das Magnetventil (140V) zwischen dem Kältemittelversorgungskanal (140I) und dem Ventilversorgungskanal (140I) vorgesehen ist, um den Fluss des gasförmigen Kältemittels gemäß einem Steuersignal zu steuern.

2. Das Radiofrequenz-Behandlungshandstück (100) nach Anspruch 1, wobei mindestens ein Durchgangsloch (111) in einer vorderen Außenwand des Handstückgehäuses (110) ausgebildet ist, und an einer Seite des ersten Körperabschnitts (141) der Elektrodenanordnung (140) ein Stufenabschnitt (141B), der in eine in dem Düsenabschnitt (130N) ausgebildete Befestigungsnut eingesetzt und verbunden ist, und ein Druckabschnitt (141P), der durch das Durchgangsloch (111) des Handstückgehäuses (110) hindurchragt, vorgesehen sind.

3. Das Radiofrequenz-Behandlungshandstück (100) nach Anspruch 1, wobei der Kältemittelversorgungskanal (140I) auf einer Mittelachse des ersten Körperabschnitts (141) befestigt ist und mit dem Ventilversorgungskanal (1401) in Verbindung steht,
wobei die Elektrodenanordnung (140) ferner umfasst: einen zweiten Körperabschnitt (143) mit einem Kanaldurchgangsloch (143H), durch das der Kältemittelversorgungskanal (140I) verläuft, und der so ausgebildet ist, dass er den ersten Aufnahmeraum (110S) und den zweiten Aufnahmeraum (140S) unterteilt; und einen zweiten Flansch (144), der sich von einem Ende des zweiten Körperabschnitts (143) nach außen erstreckt, und
wobei der Leistungsverteiler (145) in den zweiten Körperabschnitt (143) eingesetzt und daran befestigt ist.

4. Das Radiofrequenz-Behandlungshandstück (100) nach Anspruch 3, wobei der Leistungsverteiler (145) so installiert ist, dass er an dem Kältemittelversorgungskanal (140I) befestigt ist, und eine Vielzahl von Elektrodenleitungen (145E) auf einer Oberfläche des Leistungsverteilers (145) ausgebildet sind, um elektrisch mit der HF-Elektrode (131) verbunden zu sein.

5. Das Radiofrequenz-Behandlungshandstück (100) nach Anspruch 3, wobei der zweite Flansch (144) mit einer Vielzahl von Durchgangslöchern (111) an entsprechenden Positionen einer Vielzahl von Elektrodenleitungen (145E) versehen ist, und die Vielzahl von Elektrodenleitungen (145E) jeweils die Vielzahl von Durchgangslöchern (111) durchdringen und die HF-Elektrode (131) kontaktieren.

6. Das Radiofrequenz-Behandlungshandstück (100) nach Anspruch 3, wobei die Elektrodenspitze (130) ferner umfasst: einen Verteiler (132), der das von dem Kältemittelversorgungskanal (140I) zugeführte gasförmige Kältemittel gleichmäßig verteilt und das gasförmige Kältemittel der HF-Elektrode (131) zuführt; und einen Seitenauslass (133), der mit der Elektrodenanordnung (140) in Verbindung steht, so dass das gasförmige Kältemittel, das Wärme mit der HF-Elektrode (131) ausgetauscht hat, zu dem Seitenauslass (133) bewegt wird.

7. Das Radiofrequenz-Behandlungshandstück (100) nach Anspruch 6, wobei ein erster Auslass (1410) in einem Bereich des ersten Körperabschnitts (141) vorgesehen ist, um mit dem Seitenauslass (133) und dem zweiten Körperabschnitt (143) in Verbindung zu stehen, und ein zweiter Auslass in dem zweiten Körperabschnitt (143) vorgesehen ist, um mit dem ersten Auslass (1410) und dem zweiten Aufnahmeraum (140S) in Verbindung zu stehen, so dass der Kältemittel-Wärmetauschraum (130S), der zweite Aufnahmeraum (140S) und der erste Aufnahmeraum (110S) in dieser Reihenfolge miteinander verbunden sind.

## Revendications

1. Une pièce à main de traitement par radiofréquence (100), comprenant :
un boîtier de pièce à main (110) configuré pour avoir un premier espace contenant (110S) là-dedans et une carte de circuit (112) installée dans le premier espace contenant (110S) de manière à faire fonctionner la pièce à main (100), et avoir une face avant de celui-ci étant ouverte ;
une pointe d'électrode (130) configurée pour avoir une partie buse (130N) qui est couplée de manière détachable au boîtier de pièce à main (110) et pourvue d'une électrode RF (131) pour générer des ondes radiofréquences par énergie électrique appliquée à celle-ci ;
un ensemble d'électrodes (140) installé dans le premier espace contenant (110S), pourvu d'un canal d'alimentation de vanne (140I), qui est configuré pour recevoir un réfrigérant gazeux d'un moyen d'alimentation en réfrigérant, un second espace contenant (140S) séparé du premier espace contenant (110S) et un corps principal, et comprend un distributeur d'énergie (145) pour appliquer de l'énergie électrique à la pointe d'électrode (130) et une vanne solénoïde (140V) configurée pour communiquer avec le canal d'alimentation de vanne ( 1401) et pour contrôler un débit du réfrigérant gazeux ; et
un actionneur (120) installé à l'arrière du premier espace contenant (110S) et couplé à l'ensemble d'électrodes (140) de manière à faire vibrer l'ensemble d'électrodes (140) selon un signal de contrôle,
dans laquelle l'ensemble d'électrodes (140) comprend :
une première partie de corps (141) ayant une sortie arrière (141H) qui communique le premier espace contenant (110S) et le second espace contenant (140S) ;
une partie de couplage de buse (142) couplée à une surface périphérique interne de la partie buse (130N) de manière à fermer une zone ouverte située à l'intérieur de la partie buse (130N) ; et
un canal d'alimentation en réfrigérant (140I) configuré pour communiquer avec le canal d'alimentation de valve (1401) de manière à livrer un réfrigérant gazeux à la pointe d'électrode (130), et
la vanne solénoïde (140V) est pourvue entre le canal d'alimentation en réfrigérant (140I) et le canal d'alimentation de vanne (140I) de manière à contrôler un débit du réfrigérant gazeux selon un signal de contrôle.

2. La pièce à main de traitement par radiofréquence (100) de la revendication 1, dans laquelle au moins un trou traversant (111) est formé dans une paroi extérieure avant du boîtier (110) de pièce à main (100), et sur un côté de la première partie de corps (141) de l'ensemble d'électrodes (140), une partie en gradins (141B), qui s'insère et s'emboîte dans une rainure de fixation formée dans la partie buse (130N), et une partie de pressage (141P), qui fait sailli à travers le trou traversant ( 111) du boîtier de pièce à main (110), sont prévues.

3. La pièce à main de traitement par radiofréquence (100) de la revendication 1, dans laquelle le canal d'alimentation en réfrigérant (1401) est fixé sur un axe central de la première partie de corps (141) et communique avec le canal d'alimentation de vanne (140I),
l'ensemble d'électrodes (140) comprend en outre : une seconde partie de corps (143) ayant un trou traversant de canal (143H), par lequel passe le canal d'alimentation en réfrigérant (1401), et formée pour diviser le premier espace contenant (110S) et le second espace contenant (140S) ; et une seconde bride (144) s'étendant vers l'extérieur à partir d'une extrémité de la seconde partie de corps (143), et
le distributeur d'énergie (145) est inséré et fixé à la seconde partie de corps (143).

4. La pièce à main de traitement par radiofréquence (100) de la revendication 3, dans laquelle le distributeur d'énergie (145) est installé pour être fixé sur le canal d'alimentation en réfrigérant (1401), et une pluralité de fils d'électrode (145E) sont formés sur une surface du distributeur d'énergie (145) pour être connectés électriquement à l'électrode RF (131).

5. La pièce à main de traitement par radiofréquence (100) de la revendication 3, dans laquelle la seconde bride (144) est pourvue d'une pluralité de trous traversants (111) sur des positions correspondantes d'une pluralité de fils d'électrode (145E), et la pluralité de fils d'électrode (145E) pénètrent chacun la pluralité de trous traversants (111) et entrent en contact avec l'électrode RF (131).

6. La pièce à main de traitement par radiofréquence (100) de la revendication 3, dans laquelle la pointe d'électrode (130) comprend en outre : un distributeur (132) qui distribue uniformément le réfrigérant gazeux fourni par le canal d'alimentation en réfrigérant (140I) et fournit le réfrigérant gazeux à l'électrode RF (131) ; et une sortie latérale (133) qui communique avec l'ensemble d'électrodes (140), de sorte que le réfrigérant gazeux, qui a échangé de la chaleur avec l'électrode RF (131), se déplace à la sortie latérale (133).

7. La pièce à main de traitement par radiofréquence (100) de la revendication 6, dans laquelle une première sortie (1410) est pourvue dans une zone de la première partie de corps (141) pour communiquer avec la sortie latérale (133) et la seconde partie de corps (143), et une seconde sortie est prévue dans la seconde partie de corps (143) pour communiquer avec la première sortie (141O) et le second espace contenant (140S), de sorte que l'espace d'échange de chaleur de réfrigérant (130S), le second espace contenant (140S) et le premier espace contenant (110S) sont communiqués dans cet ordre.
